(19) Europäisches Patentamt
European Patent Office
Office européen des brevets

(11) Publication number: **0 074 229**
**B1**

(12) **EUROPEAN PATENT SPECIFICATION**

(45) Date of publication of patent specification: **09.04.86**

(21) Application number: **82304531.5**

(22) Date of filing: **27.08.82**

(51) Int. Cl.[4]: **C 07 D 401/04,**
C 07 D 401/14,
C 07 D 417/14,
C 07 D 405/14, A 61 K 31/00

(54) **Triazole gastric anti-secretory agents.**

(30) Priority: **31.08.81 US 298224**

(43) Date of publication of application:
**16.03.83 Bulletin 83/11**

(45) Publication of the grant of the patent:
**09.04.86 Bulletin 86/15**

(84) Designated Contracting States:
**AT BE CH DE FR GB IT LI LU NL SE**

(56) References cited:
**GB-A-2 053 910**

**GRECHISKIN et al., Farmakol. Toksikol., 39, pp. 556-60 (1976)**

**The file contains technical information submitted after the application was filed and not included in this specification**

(73) Proprietor: **PFIZER INC.**
**235 East 42nd Street**
**New York, N.Y. 10017 (US)**

(72) Inventor: **Lipinski, Christopher Andrew**
**10 Connshire Drive Waterford**
**New London Connecticut (US)**
Inventor: **LaMattina, John Lawrence**
**13 Huntington Way Ledyard**
**New London Connecticut (US)**

(74) Representative: **Wood, David John et al**
**Pfizer Limited Ramsgate Road**
**Sandwich Kent CT13 9NJ (GB)**

EP 0 074 229 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a series of novel 3'-substituted-5'-(2-amino-4-pyridyl)-1',2',4'-triazoles and their pharmaceutically acceptable salts which are antagonists of the $H_2$ histamine receptor and are therefore useful in the control of gastric secretion.

One of the known physiological effects of histamine is the stimulation of gastric acid secretion. It has been reported that a specific histamine receptor, the $H_2$ histamine receptor, mediates the stimulatory action of histamine (Black et al., Nature, 1972, *236,* 385). Accordingly, an extensive search has been undertaken for agents which block the $H_2$ receptor and thus inhibit gastric acid secretion.

Durant et al. in U.S. Nos. 3,905,984 and 4,027,026 disclose that pyridyl substituted thioalkyl, oxyalkyl thiourea and oxyalkyl urea are inhibitors of histamine activity and the $H_2$ histamine receptors. Durant et al. in U.S. Nos. 4,022,797 and 4,024,271 disclose that thioalkyl aminoalkyl and oxyalkyl quanidines are likewise inhibitors of histamine activity. It has recently been disclosed by Lipinski in U.S. Patent No. 4,276,297 that a series of novel 5'-(4-pyridyl)-1',2',4'-triazole derivatives are inhibitors of the $H_2$ histamine receptor and are useful anti-ulcer agents. In Farmakol. Toksikol., 39, 556—60 (1976) the sensitivity of histamine $H_1$ and $H_2$ receptors to derivatives of 3-(β-aminoethyl)-1,2,4-triazole is described.

The present invention relates to novel 3'-substituted-5'-(2-amino-4-pyridyl)-1',2',4'-triazoles useful as histamine $H_2$ antagonists which are anti-secretory agents and are therefore useful in the treatment of peptic ulcers and other conditions caused by or aggravated by gastric hyperacidity. More specifically, the novel compounds of this invention are those of Formula (I)

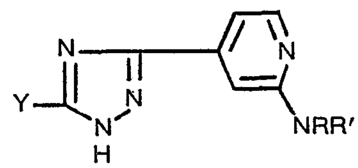

(I)

and their pharmaceutically-acceptable acid addition salts,
wherein R is $C_1$—$C_4$ alkyl; R' is hydrogen, methyl or ethyl; and
Y is hydrogen, hydroxymethyl, or $C_1$—$C_4$ alkyl.

Preferred compounds include those wherein R is methyl or ethyl, R' is hydrogen and Y is hydrogen, methyl, ethyl or hydroxymethyl.

Also embraced by the present invention are pharmaceutical compositions comprising a gastric anti-secretory effective amount of a compound of Formula (I), or a pharmaceutically acceptable salt thereof, together with a pharmaceutically acceptable carrier or diluent. Preferred pharmaceutically compositions are those containing the preferred compounds of the Formula (I) as described above.

The present invention also comprises a compound of the formula (I) or a pharmaceutically acceptable salt thereof for use in medicine, especially for use in a method of treating gastric hyperacidity in an animal in need of such treatment.

The invention also includes novel intermediates useful for the preparation of the compounds of the Formula (I) and being of the Formula (II):—

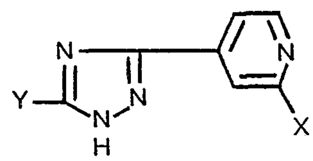

(II)

and the acid addition salts thereof wherein Y is hydrogen, alkyl of 1 to 4 carbon atoms or hydroxymethyl, and X is halo, preferably chloro.

The novel compounds of Formula (I) may be prepared by the reaction sequence shown in the Scheme. The numbering of the two heterocyclic rings in the Scheme is that employed throughout the specification.

SCHEME

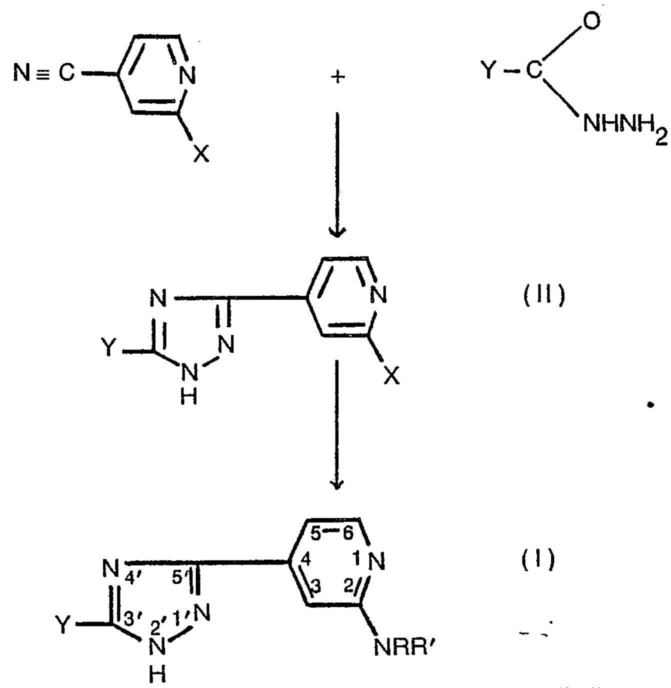

(II)

(I)

In the Scheme, a 2-haloisonicotinonitrile is treated with an alkali metal alkoxide such as sodium methoxide in an alcohol solvent such as methanol at a temperature between 15° to 35°C and then allowed to react with an essentially equimolar amount of an appropriate carboxylic acid hydrazide in a suitable solvent, preferably methanol under reflux for about 20 hours. If necessary, as in the case of the 3-hydroxymethyl derivative, the pH is maintained between 9—10 during reflux by addition of dilute base, e.g. alkali metal hydroxide preferably sodium hydroxide. It will be understood that in the resulting compound, an intermediate of Formula (II), the substituent at the 3-position is determined by the particular acid hydrazide employed in the synthesis. Thus for example acetic acid hydrazide is employed to produce 3′-methyl-5′-(2-halo-4-pyridyl)-1′,2′,4′-triazole, formic acid hydrazide to produce 3-(2-halo-4-pyridyl)-1′,2′,4′-triazole, propionic acid hydrazide to produce 3′-ethyl-5′-(2-halo-4-pyridyl)-1′,2′,4′-triazole and glycolic acid hydrazide to produce 3′-hydroxymethyl-5′-(2-halo-4-pyridyl)-1′,2′,4′-triazole.

This halo intermediate of Formula (II) is finally converted to an amino derivative of Formula (I) by heating with an appropriate aqueous alkylamine solution at about 160°C to 180°C for about 20 hours. For example, the preferred compounds of Formula (I) in which R is ethyl and R′ is hydrogen may be prepared from the halo intermediate of Formula (II) and aqueous ethylamine at about 160°C to 180°C for 16 to 18 hours.

The pharmaceutically acceptable acid addition salts of the novel compounds of the Formula (I) and the corresponding salts of the intermediates of the Formula (II) may be readily prepared by contacting the free base with an appropriate mineral or organic acid in either aqueous solution or in a suitable organic solvent. The solid salt may then be obtained by precipitation or by evaporation of the solvent. The pharmaceutically acceptable acid addition salts of this invention include, but are not limited to, the hydrochloride, sulfate, bisulfate, mesylate, tosylate, nitrate, phosphate, acetate, lactate, maleate, fumarate, citrate, tartrate, succinate, gluconate and the like. Hydrochloride salts are preferred. If desired, the compounds of Formula I and II as the free base may be formed from the acid addition salts thereof by treatment with an appropriate base followed by extraction of the free base with a suitable organic solvent.

The compounds of Formula (I) and the pharmaceutically acceptable acid addition salts thereof have activity as antisecretory agents and histamine $H_2$ antagonists and accordingly are of therapeutic value in the treatment of gastric hyperacidity and peptic ulcers. For the purposes of the present specification and claims hereof the term treatment of gastric hyperacidity is meant to include the treatment of peptic ulcers and other such conditions caused by, or aggravated, by the secretion of gastric acid. The compounds may be administered to a subject in need of treatment by a variety of conventional routes of administration including orally, intravenously and parenterally. Preferably, the compounds are administered orally. In general, these compounds will be administered orally at one or more doses between about 2 to 20 mg/kg body weight of the subject to be treated per day, preferably from about 500 to 2000 mg per day. If parenteral or intravenous administration is desired, then these compounds can be given at doses between about 1 to 20 mg/kg body weight of the subject to be treated per day. However, some variation in dosage will necessarily occur depending upon the condition of the subject being treated and the particular compound employed.

The compound may be administered alone or in combination with pharmaceutically acceptable carriers or diluents, in either single or multiple doses. Suitable pharmaceutical carriers include inert diluents or fillers, sterile aqueous solutions and various organic solvents. The pharmaceutical compositions formed by combining the novel compounds of Formula I or salts thereof and pharmaceutically acceptable carriers are readily administered in a variety of dosage forms such as tablets, powders, capsules, lozenges, syrups and the like. These pharmaceutical compositions can, if desired, contain additional ingredients such as flavorings, binders, excipients and the like. Thus, for oral administration, tablets containing various excipients, such as sodium citrate, may be employed, together with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions of elixirs are desired for oral administration, the essential active ingredient therein may be combined with various sweetening or flavoring agents, coloring matters or dyes and, if desired, emulsifying agents or suspending agents, together with diluents such as water, ethanol, propylene glycol, glycerin, or combinations thereof.

Preferably, the novel compounds of this invention are administered orally in unit dosage form, i.e. as a single physically discrete dosage unit containing an appropriate amount of the active compound in combination with a pharmaceutically acceptable carrier or diluent. Examples of such unit dosage forms are tablets or capsules containing from about 100 to 500 mg of the active ingredient, the compound of Formula I comprising from about 50% to 90% of the total weight of the dosage unit.

For parenteral administration, solutions of the compounds of Formula I in sterile aqueous solutions, for example aqueous propylene glycol, sodium chloride, dextrose or sodium bicarbonate solutions may be employed. Such solutions should be suitably buffered if necessary and the liquid diluent first rendered isotonic with sufficient saline or glucose. The preparation of suitable sterile liquid media for parenteral administration will be well known to those skilled in the art.

The activity of the compounds of the present invention as antisecretory agents and histamine-$H_2$ antagonists may be determined by standard pharmacological tests, including for example (1) measuring their ability to antagonize the actions of histamine which are not blocked by an antihistamine such as mepyramine and (2) measuring their ability to inhibit gastric acid secretion in the stomachs of Heidenhain pouch dogs that had previously been treated with pentagastrin in order to stimulate the secretion of gastric acid.

The present invention is illustrated by the following examples. However, it should be understood that the invention is not limited to the specific details of these examples. All temperatures are in degrees centigrade.

## Example 1

3'-Methyl-5'-(2-chloro-4-pyridyl)-1',2',4'-triazole

This example illustrates the preparation of an intermediate of Formula (II).

6.9 g (49.8 mmol) of 2-chloroisonicotinonitrile was stirred with sodium methoxide (5.2 mmol) from 120.2 mg of sodium in 100 ml of methanol. After one hour at 25°C, 3.7 g (49.9 mmol) of acetic acid hydrazide was added to give after several minutes a clear solution. The reaction was heated at reflux for 20 hours at which time a precipitate began to form. Reflux was continued for an additional 120 hours during which time the reaction precipitate went into solution. The reaction was cooled and the initially formed precipitate was removed by filtration. The mother liquors were concentrated in vacuo with formation of a second precipitate. This was collected by filtration and dried to give 1.8 g (19%) of 3'-methyl-5'-(2-chloro-4-pyridyl)-1',2',4'-triazole mp 215—218°C.

*Anal.* $C_8H_7ClN_4$.

| | | | | |
|---|---|---|---|---|
| Calcd: | C, 49.37; | H, 3.63; | N, 28.79; | Cl, 18.22 |
| Found: | C, 49.22; | H, 3.84; | N, 28.35; | Cl, 17.74. |

## Example 2

3'-Methyl-5'-(2-methylamino-4-pyridyl)-1',2',4'-triazole

5.3 g (27.23 mmol) of 3'-methyl-5'-(2-chloro-4-pyridyl)-1',2',4'-triazole (preparation given in Example 1) was combined with 50 ml of 40% aqueous methylamine solution in a 150 ml stainless steel reaction vessel and heated at 170°C for 21 hours. After cooling in ice, the contents of the reaction vessel were concentrated in vacuo to an orange oil from which a solid began to crystallize. The crude oily solid was slurried in ethylacetate-methanol and the resulting off-white solid was collected by filtration and dried in vacuo to give 2.1 g of crude product. This material was taken up and recrystallized from ethylacetate-methanol. Upon concentration and cooling 211 mg of material mp 294—295°C was collected. On standing the mother liquors deposited 395 mg (7%) of 3'-methyl-5'-(2-methylamino-4-pyridyl)-1',2',4'-triazole, mp 208—210°C. NMR ($Me_2SO$) δ 7.98 (d, 1H), 7.05 (m, 3H), 2.86 (broad s, 3H), 2.42 (s, 3H).

Example 3

3'-Methyl-5'-(2-ethylamino-4-pyridyl)-1',2',4'-triazole

1.6 g (8.22 mmol) of 3'-methyl-5'-(2-chloro-4-pyridyl)-1',2',4'-triazole (preparation given in Example 1) was combined with 50 ml of 70% aqueous ethylamine and placed in a 150 ml stainless steel reaction vessel and heated at 170°C for 16.5 hours. Using a similar procedure, 4.4 g (22.61 mmol) of 3'-methyl-5'-(2-chloro-4-pyridyl)-1',2',4'-triazole was combined with 50 ml of 70% aqueous ethylamine and heated at 170°C for 16.5 hours. On cooling, the contents of the reaction vessels were combined and concentrated in vacuo to a crude brown solid. Water was removed by repeatedly concentrating in vacuo with methanol to give a crude brown solid. This material was crystallized from acetonitrile-ethanol to give a pale brown solid mp 223—227°C. This material was recrystallized from acetonitrile-ethanol to give 1.636 g (26%) of 3'-methyl-5'-(2-ethyl-amino-4-pyridyl)-1',2',4'-triazole as an off-white solid, mp 226—228°C; NMR ($Me_2SO$) δ 8.03 (1H, d), 7.03 (2H, m), 6.57 (1H, t), 3.33 (2H, m), 2.43 (3H, s), 1.17 (3H, t).

*Anal.* $C_{10}H_{13}H_5$.

| | | | |
|---|---|---|---|
| Calcd: | C, 59.09; | H, 6.45; | N, 34.46. |
| Found: | C, 58.76; | H, 6.42; | N, 34.48. |

Example 4

3'-(2-Chloro-4-pyridyl)-1',2',4'-triazole hydrate

This example illustrates the preparation of an intermediate compound of formula (II).

6.9 g (49.8 mmol) of 2-chloroisonicotinonitrile was stirred with a catalytic amount of sodium methoxide (3.8 mol) from 86.4 mg of sodium in 100 ml methanol. Within 5 minutes all the isonicotinonitrile was in solution. After one hour at 25°C, 3.0 g (49.9 mmol) of formic acid hydrazide was added to give a clear solution. The solution was heated at reflux. After three hours a precipitate began to form. Reflux was continued for a total of 24 hours. The reaction was cooled and a yellow solid collected by filtration. The mother liquors were concentrated and a second solid precipitated. This material was collected to give 4.9 g (50%) of 3'-(2-chloro-4-pyridyl)-1',2',4'-triazole hydrate, mp 184—186°C.

*Anal.* $C_7H_5ClN_4 \cdot H_2O$,

| | | | |
|---|---|---|---|
| Calcd: | C, 42.33; | H, 3.55; | N, 28.21. |
| Found: | C, 42.53; | H, 3.74; | N, 28.37. |

Example 5

3'-(2-Ethylamino-4-pyridyl)-1',2',4'-triazole

4.8 g (26.58 mmol) of 3'-(2-chloro-4-pyridyl)-1',2',4'-triazole (preparation given in Example 4) was combined with 70 ml of 70% aqueous ethylamine and placed in a 150 ml stainless steel reaction vessel and heated at 170° for 18 hours. After cooling, the contents of the vessel were filtered and the filtrate evaporated to an orange oil. This material was chromatographed on 400 g silica gel using ethyl acetate as eluent to give 2.158 g (43%) of 3'-(2-ethylamino-4-pyridyl)-1',2',4'-triazole as a creme colored solid, mp 153—155°C; NMR ($Me_2SO$) δ 8.38 (s, 1H), 7.93 (d, 1H), 6.95 (m, 2H), 6.53 (t, 1H), 3.25 (m, 2H), 1.13 (t, 3H).

*Anal.* $C_9H_{11}N_5$,

| | | | |
|---|---|---|---|
| Calcd: | C, 57.12; | H, 5.86; | N, 37.02. |
| Found: | C, 56.56; | H, 5.84; | N, 36.58. |

Example 6

3'-Ethyl-5'-(2-ethylamino-4-pyridyl)-1',2',4'-triazole hemi-hydrate

5.0 g (36.08 mmol) of 2-chloroisonicotinonitrile was stirred in 100 ml of $CH_3OH$ with a catalytic amount of sodium methoxide (5.39 mmol) from 124 mg of sodium. After stirring for one hour at 25°C, 6.0 g (31.9 mmol) of propionic acid hydrazide was added and the reaction was heated at reflux for 40 hours. An additional portion of 3.3 g (17.5 mmol) of propionic acid hydrazide was added and reflux was continued for an additional 120 hours. The reaction was cooled and concentrated in vacuo to an orange oil which was triturated with an ether-ethylacetate-methanol mixture to remove a small quantity of yellow solid lacking an ultraviolet chromophore. The mother liquor was reconcentrated in vacuo to an orange oil and combined with 75 ml of 70% aqueous ethylamine and placed in a 150 ml stainless steel reaction vessel and heated at 160° for 17 hours. After cooling, the reaction contents were concentrated in vacuo to a green oil which was triturated with an ethylacetate methanol mixture to remove a solid with no ultraviolet absorbance. The mother liquors were reconcentrated to an oil and chromatographed on silica gel using a 95:5 mixture of ethylacetate-methanol as eluent. In this manner was obtained 214.1 mg (2.6%) of 3-ethyl-5-(2-ethylamino-4-pyridyl)-1',2',4'-triazole hemihydrate, mp 142—144°C; NMR ($CDCl_3$) δ 9.3 (broad s, 1H) 7.8 (d, 1H), 7.53—7.2 (m, 3H), 3.53 (s, 1H, 1/2 $H_2O$), 3.41 (m, 2H), 2.91 (q, 2H), 1.40 (t, 3H), 1.13 (t, 3H).

*Anal.* $C_{11}H_{15}N_5 \cdot 1/2\ H_2O$,

| | | | |
|---|---|---|---|
| Calcd: | C, 58.38; | H, 7.13; | N, 30.95. |
| Found: | C, 58.07; | H, 6.89; | N, 30.62. |

Example 7

3′-Hydroxymethyl-5′-(2-chloro-4-pyridyl)-1′,2′,4′-triazole

This example illustrates the preparation of an intermediate compound of Formula (II).

23.5 g (169.6 mmol) of 2-chloroisonicotinonitrile was dissolved in 300 ml of methanol containing 21.7 mmol of sodium methoxide prepared from 500 mg sodium. After stirring one hour at 25°C, 15.3 g (169.6 mmol) of glycolic acid hydrazide was added and the reaction was heated at reflux for 2.5 hours. After cooling, the reaction was concentrated in vacuo and the methanol solvent was replaced by a solution of 1:1 dioxane-water. The reaction was heated to reflux and dilute NaOH solution was added periodically to maintain the pH at 9 to 10 over a period of 6 hours. While still hot, the reaction was filtered and the clear filtrate allowed to remain at 25°C over 60 hours. During this time there formed a precipitate of 17.6 g (49%) 3′-hydroxymethyl-5′-(2-chloro-4-pyridyl)-1′,2′,4′-triazole, mp 166—168°C. High resolution mass spectrum $C_8H_7N_4OCl$, $Cl^{37}$ isotope M/E Calcd. 212.0278.
Found: 212.0274. $Cl^{35}$ isotope M/E Calcd. 210.0308.
Found: 210.0311. NMR ($Me_2SO$) δ 8.43 (d, 1H), 7.87 (m, 2H), 4.67 (s, 2H).

*Anal.* $C_8H_7ClN_4O$,

| | | | |
|---|---|---|---|
| Calcd: | C, 45.62; | H, 3.35; | N, 26.60. |
| Found: | C, 45.70; | H, 3.67; | N, 26.55. |

Example 8

3′-Hydroxymethyl-5′-(2-ethylamino-4-pyridyl)-1′,2′,4′-triazole

5.0 g (23.74 mmol) of 3′-hydroxymethyl-5′-(2-chloro-4-pyridyl)-1′,2′,4′-triazole (preparation given in Example 7) was combined with 100 ml of 50% aqueous ethylamine solution and heated at 180°C for 18 hours in a stainless steel reaction vessel. After cooling the contents were concentrated in vacuo and triturated with a methanol-ethylacetate mixture to give 3.6 g of a beige colored solid. This was recrystallized from methanol-ethylacetate to give 1.9 g (36%) of 3′-hydroxymethyl-5′-(2-ethylamino-4-pyridyl)-1′,2′,4′-triazole, mp 226—228°C; NMR ($Me_2SO$) δ 7.93 (d, 1H), 6.97 (m, 2H) 6.47 (t, 1H), 4.6 (s, 2H), 3.27 (m, 2H), 2.83 (t, 3H).

*Anal.* $C_{10}H_{13}N_5O$,

| | | | |
|---|---|---|---|
| Calcd: | C, 54.78; | H, 5.98; | N, 31.95. |
| Found: | C, 54.58; | H, 6.15; | N, 31.49. |

Example 9

The gastric antisecretory activity of compounds of the present invention was determined in overnight fasted, conscious Heidenhain pouch dogs. Pentagastrin (Peptavlon-Ayerst) was used to stimulate acid output by continuous infusion into a superficial leg vein at doses earlier determined to stimulate near maximal acid output from the gastric pouch. Gastric juice was collected at 30 minute intervals following the start of a pentagastrin infusion and measured to the nearest 0.1 ml. Ten collections were taken for each dog during an experiment. Acid concentration was determined by titrating 1.0 ml of gastric juice to pH 7.4 with 0.1*N* sodium hydroxide using an Autoburette and a glass electrode pH meter (Radiometer).

Drug or vehicle was given intravenously 90 minutes following the start of the pentagastrin infusion, at a dose of 1 mg/kg. Gastric acid antisecretory effects were calculated by comparing the lowest acid output after drug administration with the mean acid output immediately before drug.

The results obtained are as follows:

Compounds of Examples #2, 3 and 8 gave greater than 50% inhibition of acid secretion in dogs at doses of 10 mg/kg or less.

Example 10

The histamine-$H_2$ antagonist activity of compounds of the present invention was determined by the following procedure.

Guinea pigs are killed rapidly with a blow to the head, the heart removed and the right atria dissected free. Atria are suspended, isometrically, in a temperature-controlled (32°±2°) tissue bath (10 ml) containing oxygenated (95% $O_2$; 5% $CO_2$) Krebs-Henseleit buffer (pH 7.4) and are allowed to stabilize approximately one hour during which time the tissue bath is flushed several times. Individual atrial contractions are followed with a force-displacement transducer connected to a cardiotachometer and Grass polygraph recorder. After obtaining a dose-response curve to histamine, the bath containing each atrium is flushed several times with fresh buffer and the atria re-equilibrated to basal rates. Following the return to basal

rate, test compounds are added at selected final concentrations and the histamine dose-response curve is again determined in the presence of antagonist. Results are expressed as dose-ratios, the ratio of histamine concentrations required to produce one-half of maximal stimulation in the presence and absence of antagonist, and the apparent dissociation constant of the $H_2$-receptor antagonist $pA_2$ is determined. The results obtained are as follows:

1. Compounds of Examples #2, 3 and 6 gave $pA_2$ values of 6.0 or greater.
2. Compounds of Examples #5 and 8 gave $pA_2$ values between 5.0 and 6.0.

**Claims for the Contracting States: BE CH DE FR GB IT LI LU NL SE**

1. A compound of the formula

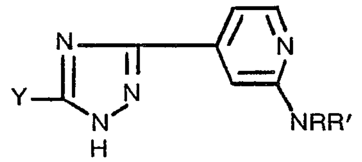

(I)

or a pharmaceutically acceptable acid addition salt thereof, wherein R is $C_1$—$C_4$ alkyl; R′ is hydrogen, methyl or ethyl; and

Y is hydrogen, hydroxymethyl or $C_1$—$C_4$ alkyl.

2. A compound of claim 1 wheein R is ethyl or methyl, R′ is hydrogen and Y is hydrogen, methyl, ethyl or hydroxymethyl.

3. A pharmaceutical composition comprising a compound of the formula (I) as claimed in either of the preceding claims, or a pharmaceutically acceptable acid addition salt thereof, together with a pharmaceutically acceptable diluent or carrier.

4. A compound of the formula (I) as claimed in either of claims 1 and 2, or a pharmaceutically acceptable acid addition salt thereof, for use in treating a condition caused or aggravated by hyperacidity in a human being.

5. A process for preparing a compound of the formula (I) as defined in claim 1 or a pharmaceutically acceptable acid addition salt thereof, characterised in that a compound of the formula:—

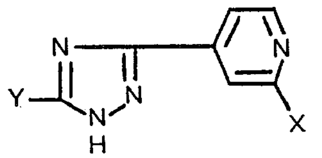

wherein X is halo and Y is as defined above,

is contacted with an alkylamine of the formula HNRR′ where R and R′ are as defined above, followed by, optionally, conversion of the compound of formula (I) into a pharmaceutically acceptable acid addition salt.

6. A process as claimed in claim 5, which is carried out in aqueous media and wherein X is Cl.

7. A compound of the formula:—

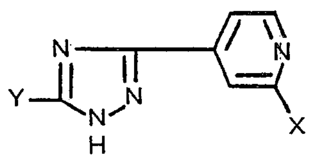

(II)

or an acid addition salt thereof,
wherein X is halo and Y is as defined in claim 1.

8. A compound as claimed in claim 7 wherein X is Cl.

**Claims for the Contracting State: AT**

1. A process for preparing a compound of the formula

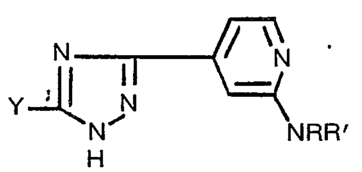

(I)

or a pharmaceutically acceptable acid addition salt thereof, wherein Y is hydrogen, hydroxymethyl or $C_1$—$C_4$ alkyl; R is $C_1$—$C_4$ alkyl; and R' is hydrogen, methyl or ethyl;

characterised in that a compound of the formula:—

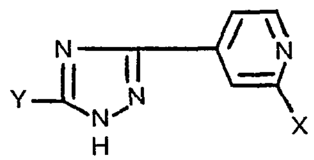

(II)

wherein X is halo and Y is as defined above,

is contacted with an alkylamine of the formula NHRR' where R and R' are as defined above, followed by, optionally, conversion of the compound of formula (I) into a pharmaceutically acceptable acid addition salt.

2. A process as claimed in claim 1, which is carried out in aqueous media and wherein X is Cl.

3. A process for preparing a pharmaceutical composition characterised by mixing a compound of the formula (I) as defined in claim 1 or a pharmaceutically acceptable acid addition salt thereof, with a pharmaceutically acceptable diluent or carrier.

4. A process for preparing a compound of the formula (II) as defined in claim 1, characterised by reacting a compound of the formula:—

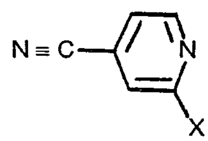

with a compound of the formula:—

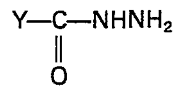

where X and Y are as defined in claim 1.

**Patentansprüche für die vertragstaaten: BE CH DE FR GB IT LI LU NL SE**

1. Verbindung mit der Formel

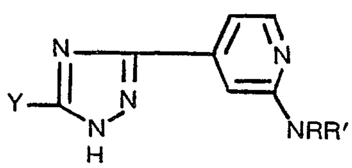

(I)

oder ein pharmazeutisch annehmbares Säureadditions-Salz hiervon, worin R $C_1$—$C_4$-Alkyl ist, R' Wasserstoff, Methyl oder Ethyl ist, und

Y Wasserstoff, Hydroxymethyl oder $C_1$—$C_4$-Alkyl ist.

2. Verbindung nach Anspruch 1, worin R Ethyl oder Methyl ist, R' Wasserstoff ist und Y Wasserstoff, Methyl, Ethyl oder Hydroxymethyl ist.

3. Pharmazeutische Zusammensetzung, die eine Verbindung mit der Formel (I), wie in einem der beiden vorangehenden Ansprüche beansprucht, oder ein pharmazeutisch annehmbares Säureadditions-Salz hiervon zusammen mit einem pharmazeutische annehmbaren Verdünnungsmittel oder Träger enthält.

4. Verbindung mit der Formel (I) wie in jedem der beiden Ansprüche 1 und 2 beansprucht, oder ein pharmazeutisch annehmbares Säureadditions-Salz hiervon zur Verwendung bei der Behandlung eines Leidens, das in einem Menschen durch Übersäuerung verursacht oder verschlimmert worden ist.

5. Verfahren zur Herstellung einer Verbindung mit der Formel (I) wie in Anspruch 1 definiert, oder ein pharmazeutisch annehmbares Säureadditions-Salz hiervon, dadurch gekennzeichnet, daß man eine Verbindung mit der Formel

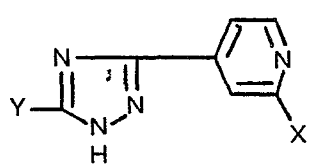

worin X Halogen und Y wie oben definiert ist, mit einem Alkylamin mit der Formel HNRR', worin R und R' wie oben definiert sind, in Kontakt bringt, fakultativ gefolgt von Umwandeln der Verbindung mit der Formel (I) in ein pharmazeutisch annehmbares Säureadditions-Salz.

6. Verfahren nach Anspruch 5, das in wäßrigem Medium durchgeführt wird und worin X gleich Cl ist.

7. Verbindung mit der Formel:

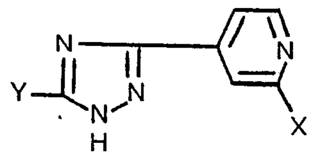

(II)

oder ein Säureadditions-Salz hiervon,
worin X Halogen und Y wie in Anspruch 1 definiert ist.

8. Verbindung wie in Anspruch 7 beansprucht, worin X gleich Cl ist.

**Patentansprüche für den Vertragsstaat: AT**

1. Verfahren zur Herstellung einer Verbindung mit der Formel

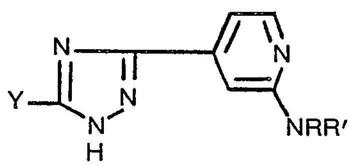

(I)

oder eines pharmazeutisch annehmbaren Säureadditions-Salzes hiervon, worin Y Wasserstoff, Hydroxymethyl oder $C_1$—$C_4$-Alkyl ist, R $C_1$—$C_4$-Alkyl ist und R' Wasserstoff, Methyl oder Ethyl ist, dadurch gekennzeichnet, daß man eine Verbindung mit der Formel:

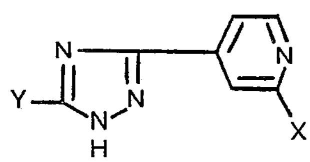

(II)

worin X Halogen und Y wie oben definiert ist,
mit einem Alkylamin mit der Formel NHRR', worin R und R' wie oben definiert sind, in Kontakt bringt, fakultativ gefolgt von Umwandeln der Verbindung mit der Formel (I) in ein pharmazeutisch annehmbares Säureadditions-Salz.

2. Verfahren wie in Anspruch 1 beansprucht, das in wäßrigem Medium durchgeführt wird und worin X gleich Cl ist.

3. Verfahren zur Herstellung einer pharmazeutischen Zusammensetzung, dadurch gekennzeichnet, daß man eine Verbindung mit der Formel (I) wie in Anspruch 1 definiert oder ein pharmazeutisch annehmbares Säureadditions-Salz hiervon mit einem pharmazeutisch annehmbaren Verdünnungsmittel oder Träger vermischt.

4. Verfahren zur Herstellung einer Verbindung mit der Formel (II) wie in Anspruch 1 definiert, dadurch gekennzeichnet, daß man eine Verbindung mit der Formel:

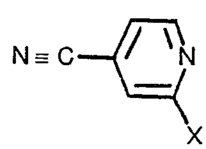

mit einer Verbindung mit der Formel:

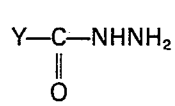

worin X und Y wie in Anspruch 1 definiert sind, zur Reaktion bringt.

**Revendications pour les Etats contractants: BE CH DE FR GB IT LI LU NL SE**

1. Composé de formule

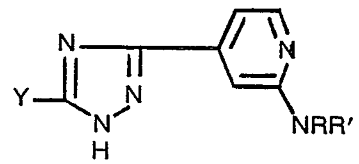

(I)

ou un sel d'addition d'acide pharmaceutiquement acceptable de ce composé, formule dans laquelle R est un groupe alkyle en $C_1$ à $C_4$; R' représente l'hydrogène, le groupe méthyle ou le groupe éthyle; et Y est l'hydrogène, le groupe hydroxyméthyle ou un groupe alkyle en $C_1$ à $C_4$.

2. Composé suivant la revendication 1, dans lequel R est le groupe éthyle ou méthyle, R' est l'hydrogène et Y est l'hydrogène, le groupe méthyle, éthyle ou hydroxyméthyle.

3. Composition pharmaceutique, comprenant un composé de formule (I) suivant l'une quelconque des revendications précédentes ou un sel d'addition d'acide pharmaceutiquement acceptable de ce composé, en association avec un diluant ou support acceptable du point de vue pharmaceutique.

4. Composé de formule (I) suivant l'une quelconque des revendications 1 et 2 ou un sel d'addition d'acide pharmaceutiquement acceptable de ce composé, destiné à être utilisé dans le traitement d'un état provoqué ou aggravé par une hyperacidité chez un être humain.

5. Procédé de préparation d'un composé de formule (I) suivant la revendication 1 ou d'un sel d'addition d'acide pharmaceutiquement acceptable de ce composé, caractérisé en ce qu'un composé de formule:

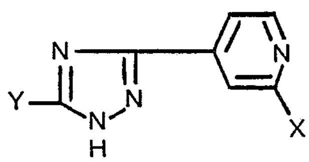

dans laquelle X est un radical halogéno et Y a la définition donnée ci-dessus,

est mis en contact avec une alkylamine de formule NHRR' dans laquelle R et R' sont tels que définis ci-dessus, la mise en contact étant suivie à titre facultatif, de la transformation du composé de formule (I) en un sel d'addition d'acide pharmaceutiquement acceptable.

6. Procédé suivant la revendication 5, qui est mis en oeuvre dans un milieu aqueux et dans lequel X représente Cl.

7. Composé de formule:

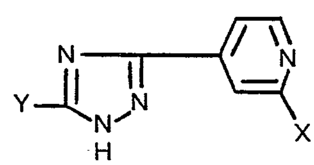

(II)

ou un sel d'addition d'acide de ce composé, formule dans laquelle X est un radical halogéno et Y a la définition donnée dans la revendication 1.

8. Composé suivant la revendication 7, dans lequel X représente Cl.

**Revendications pour l'Etat contractant: AT**

1. Procédé de préparation d'un composé de formule

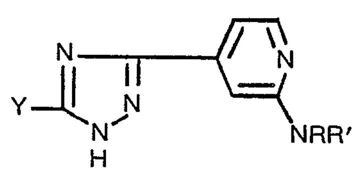

(I)

ou d'un sel d'addition d'acide pharmaceutiquement acceptable de ce composé, formule dans laquelle Y représente l'hydrogène, le groupe hydroxyméthyle ou un groupe alkyle en $C_1$ à $C_4$; R est un groupe alkyle en $C_1$ à $C_4$; et R' représente l'hydrogène, le groupe méthyle ou le groupe éthyle; caractérisé en ce qu'un composé de formule:

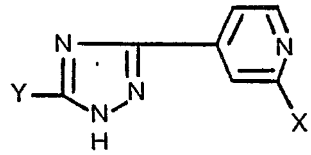

(II)

dans laquelle X est un radical halogèno et Y a la définition donnée ci-dessus,

est mis en contact avec une alkylamine de formule NHRR′ dans laquelle R et R′ sont tels que définis ci-dessus, la mise en contact étant suivie, à titre facultatif, de la transformation du composé de formule (I) en un sel d'addition d'acide pharmaceutiquement acceptable.

2. Procédé suivant la revendication 1, qui est mis en oeuvre en milieu aqueux et dans lequel X représente Cl.

3. Procédé de préparation d'une composition pharmaceutique, caractérisé en ce qu'on mélange un composé de formuled (I) tel que défini dans la revendication 1 ou un sel d'addition d'acide pharmaceutiquement acceptable de ce composé, avec un diluant ou support acceptable du point de vue pharmaceutique.

4. Procédé de préparation d'un composé de formula (II) tel que défini dans la revendication 1, caractérisé par la réaction d'un composé de formule:

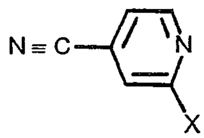

avec un composé de formule:

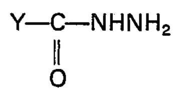

où X et Y sont tels que définis dans la revendication 1.